# EUROPEAN PATENT APPLICATION

(11) **EP 3 009 063 A1**
(43) Date of publication of application: **20.04.2016**
(21) Application number: 15194475.8
(22) Date of filing: 28.12.2011
(51) Int. Cl.: A61B 1/05, A61B 1/00, H04N 5/225

(54) **ELECTRONIC ENDOSCOPE HAVING IMAGING DEVICE**

(30) Priority: 31.01.2011 JP 2011018403
(62) Divisional of application: 11195907.8
(71) Applicant: Fujifilm Corporation, Minato-ku Tokyo 106-8620 (JP)
(72) Inventor: KIMURA, Soichiro, Kanagawa (JP)
(74) Representative: Klunker . Schmitt-Nilson . Hirsch

(57) **Abstract**

An end portion of a signal cable (37) is fixed to the inside of a rear end (40e) of a connecting member (40) by a first adhesive material (42). A flange (26a) of a lens barrel (26) is engaged to a pair of claws (40d) of the connecting member (40). The signal cable (37) is connected to the lens barrel (26) through the connecting member (40) so that the signal cable (37) is not peeled from a flexible substrate (35). The connecting member (40) does not cover a CCD (29) and a first tip (35a) of the flexible substrate (35). The diameter of a distal portion (14) of an electronic endoscope (10) can be made smaller by eliminating the thickness of the connecting member (40) and the thickness of the adhesive material (42) filled between the connecting member (40) and the CCD (29).

## Description

### BACKGROUND OF THE INVENTION

### 1. Field of the Invention

The present invention relates to an imaging device and an electronic endoscope having the imaging device.

### 2. Description Related to the Prior Art

An electronic endoscope is provided with an insertion section that is introduced into a patient's body and an operation section for operating the insertion section. The insertion section is constituted of a hard distal portion provided at its distal end side, a bending portion for directing the distal portion in a desired direction, and a flexible portion that exists between the bending portion and the operation section. The flexible portion is approximately 1 m to 2 m depending on the intended use of the electronic endoscope.

An optical system and an imaging device are incorporated in the distal portion. The optical system is constituted of a plurality of optical components such as a lens and a prism. The imaging device is constituted of a solid state imaging element such as a CCD for photoelectrically converting an optical image formed by the optical system into an imaging signal. The solid state imaging element is connected to a signal cable through a flexible substrate, and the signal cable is electrically connected to an image processing device. In addition, electronic components are mounted on the flexible substrate for driving the solid state imaging element. The imaging signal outputted from the imaging device is subjected to appropriate signal processing in the image processing device, and thereby displaying an image of a lesion and the like on a monitor.

Since the signal cable is inserted throughout the length of the insertion section, the signal cable is strongly pushed and pulled every time the insertion section is looped or the bending portion of the insertion section is largely bent. Because of this, the signal cable is sometimes peeled from the flexible substrate.

To avoid such peeling, various techniques are proposed. For example, in an electronic endoscope disclosed in Japanese Patent Laid-Open Publication No. 5-261064, a signal cable is soldered to one end of a flexible substrate. The flexible substrate is bent into a channel shape so as to surround the soldered signal cable. Moreover, a periphery of the soldered part is covered with a shield tape and an insulating tape, and an internal space formed therein is filled with an epoxy adhesive material to be fixed. Furthermore, the circuit substrate on the side where the signal cable is fixed is secured to a connection barrel by a fixation screw through a retention plate. Owing to this, the circuit substrate does not move even if the signal cable is strongly pushed and pulled, and therefore forces of twist and tilt applied from the signal cable to the circuit substrate are absorbed by the flexible circuit substrate. Therefore, such forces are not transmitted to a solid state imaging element and an objective lens system.

In an imaging device disclosed in Japanese Patent Laid-Open Publication No. 9-146011, a connection part of a flexible substrate and a signal cable is covered with a seal material to be fixed.

In an imaging device disclosed in Japanese Patent Laid-Open Publication No. 2008-118568, a reinforcing frame for housing a solid state imaging element and an electronic component mounting section of a flexible substrate is provided, and an adhesive material is filled inside the reinforcing frame. Moreover, a front end of a signal cable being soldered to the flexible substrate and the reinforcing frame are covered with a heat shrinkable tube, and the inside thereof is filled with the adhesive material to be sealed.

In the imaging device disclosed in the Japanese Patent Laid-Open Publication No. 5-261064, the circuit substrate needs to be secured to the connection barrel using the fixation screw, which is cumbersome. In the imaging device disclosed in the Japanese Patent Laid-Open Publication No. 9-146011, the forces which the signal cable is applied when being pushed and pulled are transmitted to the connection part with the flexible substrate or to the flexible substrate itself. The forces transmitted to the flexible substrate are applied to the soldered part of the signal cable and the flexible substrate or to the connection part of the flexible substrate and the solid state imaging element, which may cause peeling or breakage at any part with low resistance.

In the imaging device disclosed in the Japanese Patent Laid-Open Publication No. 2008-118568, since the solid state imaging element is housed in the reinforcing frame, the size of the solid state imaging element has an influence on the size of the reinforcing frame. As the need for higher quality of captured images is increasing every year, the solid state imaging element must be upsized. However, if the solid state imaging element is upsized, the reinforcing frame for housing the solid state imaging element also needs to be upsized. Owing to this, the diameter of the distal portion of the insertion section is made larger, which increases burdens on the patient.

### SUMMARY OF THE INVENTION

An object of the present invention is to provide an imaging device that can prevent peeling and breakage of components such as a signal cable, a flexible substrate, a solid state imaging element and connection parts thereof even when the signal cable is strongly pushed and pulled, and an electronic endoscope that can decrease burdens on a patient by making a diameter of a distal portion of the electronic endoscope small.

To achieve the above and other objects, an imaging device of the present invention includes a flexible substrate, a signal cable, and a connecting member. At a first tip of the flexible substrate, a solid state imaging element that photoelectrically converts an image formed by an objective lens system is mounted. The objective lens system is housed in a lens barrel. An optical axis of the objective lens system is bent toward the solid state imaging element by a prism. The signal cable is electrically connected to the flexible substrate. The connecting member connects the lens barrel and the signal cable. The connecting member does not cover at least one surface of the prism, the solid state imaging element, and the first tip of the flexible substrate.

The prism is in the shape of a right triangle having a tilted surface as a reflection surface. The one surface is the tilted surface.

It is preferable that the connecting member has a channel-like portion for covering at least an end portion of the signal cable and a part of the flexible substrate from three sides. The end portion of the signal cable is preferably fixed to a narrow end of the channel-like portion.

It is preferable that a first resin and a second resin are included. The first resin is filled inside the channel-like portion of the connecting member for sealing at least a part of the prism and a part of the flexible substrate. The second resin seals the prism and a part of the flexible substrate except the parts being sealed by the first resin.

The second resin is preferably applied to at least a periphery of the solid state imaging element.

It is also preferable that a first resin, a second resin and a third resin are included. The first resin seals the end portion of the signal cable. The second resin seals a part of the flexible substrate where the signal cable is connected. The third resin seals a part of the flexible substrate except the parts being sealed by the first resin and the second resin and a part of the prism.

After being hardened, the hardness of the second resin is preferably lower than the hardness of the first resin. In addition, the hardness of the second resin and the third resin are preferably almost equal and is lower than the hardness of the first resin.

It is also preferable that a first resin, a second resin, a third resin and a fourth resin are included. The first resin seals the end portion of the signal cable. The second resin seals a part of the prism and a part of the flexible substrate where the signal cable is connected. The third resin seals a part of the flexible substrate except the parts being sealed by the first resin and the second resin. The fourth resin seals a periphery of a part where the lens barrel and the prism are in contact.

After being hardened, it is preferable that the hardness of the first resin, the second resin and the third resin are equally high, and the hardness of the fourth resin is lower than the hardness of the first resin, the second resin and the third resin.

It is also preferable that a first resin, a second resin, a third resin and a fourth resin are included. The first resin seals the end portion of the signal cable. The second resin seals a part of the flexible substrate where the signal cable is connected. The third resin seals a part of the prism, a part of the lens barrel near the prism and a part of the flexible substrate except the part being sealed by the second resin. The fourth resin seals a part of the prism and a part of the flexible substrate except the parts being sealed by the second resin and the third resin.

After being hardened, it is preferable that the hardness of the second resin is lower than the hardness of the first resin, and the hardness of the third resin and the fourth resin are almost equal to the hardness of the first resin.

An electronic endoscope of the present invention includes an insertion section in which one of the above-described imaging device is incorporated at a distal portion of the insertion section.

According to the present invention, the lens barrel and the signal cable are connected by the connecting member, and thereby covering at least one surface of the prism. Owing to this, peeling and breakage of the components such as the signal cable, the flexible substrate, the solid state imaging element and the connection parts thereof can be prevented even when the signal cable is strongly pushed and pulled. Moreover, since the first tip of the flexible substrate where the solid state imaging element is mounted and the solid state imaging element are not covered by the connecting member, the diameter of the distal portion of the electronic endoscope can be made small, which decreases burdens on the patient.

### BRIEF DESCRIPTION OF THE DRAWINGS

For more complete understanding of the present invention, and the advantage thereof, reference is now made to the following descriptions taken in conjunction with the accompanying drawings, in which:
Fig. 1 is an external view illustrating an electronic endoscope of the present invention;
Fig. 2 is a sectional view illustrating a distal portion of the electronic endoscope provided with an imaging device according to a first embodiment of the present invention;
Fig. 3 is a perspective view illustrating an example of a flexible substrate;
Fig. 4 is a perspective view illustrating the imaging device;
Fig. 5 is a sectional view illustrating an imaging device according to a second embodiment;
Fig. 6 is a sectional view illustrating an imaging device according to a third embodiment;
Fig. 7 is a sectional view illustrating an imaging device according to a fourth embodiment;
Fig. 8 is a sectional view illustrating an imaging device according to a fifth embodiment;
Fig. 9 is a sectional view illustrating an imaging device according to a sixth embodiment;
Fig. 10 is a perspective view illustrating another example of a flexible substrate;
Fig. 11 is a perspective view illustrating yet another example of a flexible substrate;
Fig. 12 is a perspective view illustrating another example of a connecting member;
Fig. 13 is a sectional view illustrating an imaging device using the connecting member shown in Fig. 12; and
Fig. 14 is a perspective view illustrating an imaging device using yet another connecting member.

### DESCRIPTION OF THE PREFERRED EMBODIMENTS

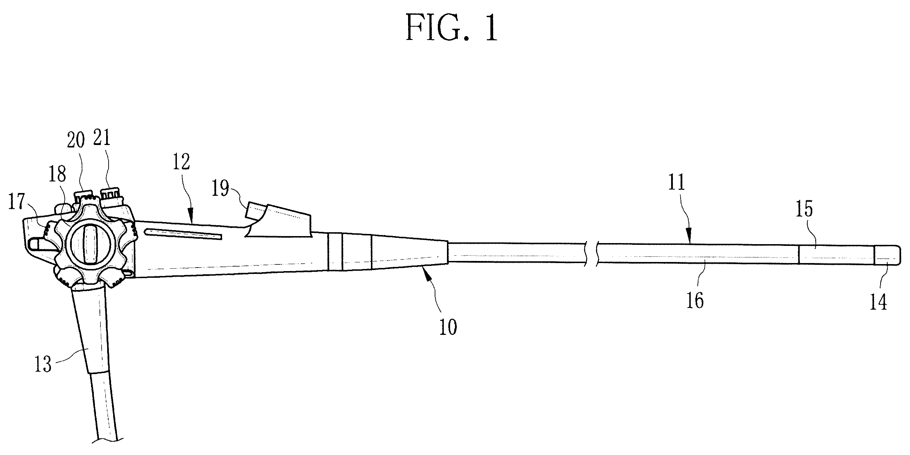

As shown in Fig. 1, an electronic endoscope 10 is provided with an insertion section 11, an operation section 12, and a universal cord 13. The insertion section 11 is introduced into a patient's body. The operation section 12 is connected to a base end portion of the insertion section 11. The universal cord 13 is connected to a processing device and a light source device (both not shown).

The processing device applies various types of image processing to an imaging signal from the electronic endoscope 10 through the universal cord 13 to convert the imaging signal into a video signal, and then displays the video signal as an endoscope image on a monitor (not shown).

The insertion section 11 is constituted of a hard distal portion 14, a bending portion 15, and a flexible portion 16 in a sequence in this order from a distal end. Inside the distal portion 14 is incorporated an imaging device and the like. The bending portion 15 is constituted of a plurality of joint pieces connected in series. An angle wire (not shown) that is inserted through the flexible portion 16 is pulled by operating angle knobs 17 and 18, and the bending portion 15 is bent up and down and to the right and left in accordance with the movement of the angle wire. Owing to this, the distal portion 14 is directed to a desired direction inside a body cavity, and a site to be observed inside the body cavity can be captured with the imaging device. The rod-like flexible portion 16 has a small diameter and connects the operation section 12 and the bending portion 15.

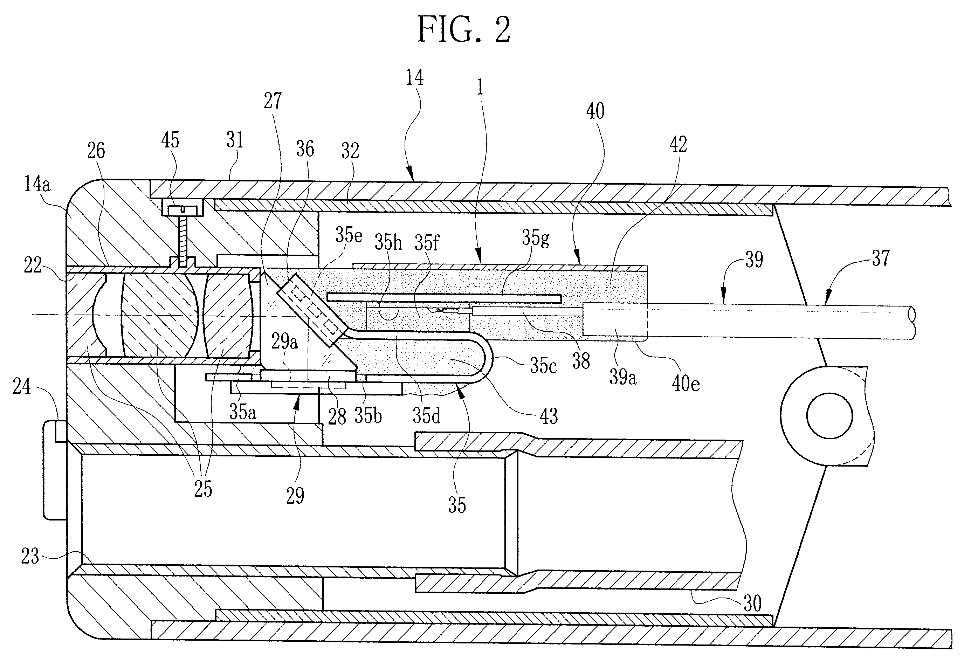

The operation section 12 is provided with a medical instrument insertion port 19. A medical instrument such as a forceps or a needle used for a treatment of affected parts is inserted through the medical instrument insertion port 19. Through a medical instrument channel 30 (see Fig. 2) disposed inside the insertion section 11, the medical instrument insertion port 19 is connected to a medical instrument outlet 23 (see Fig. 2) provided at the distal portion 14.

The operation section 12 is provided with an air/water supply button 20 and a suction button 21. An air/water nozzle 24 (see Fig. 2) formed at an end surface of the distal portion 14 supplies air or liquid like water through an air/water channel (not shown) provided inside the insertion section 11 in response to the operation of the air/water supply button 20. The suction button 21 is operated when suctioning body fluid, tissue or the like from the inside of the patient's body through the medical instrument outlet 23.

In Fig. 2, an image capturing window 22, an illumination window (not shown), the medical instrument outlet 23, and the air/water nozzle 24 are formed at the end surface of the distal portion 14. Behind the image capturing window 22, an objective lens system (imaging lens) 25 for taking image light inside the body cavity is disposed. The objective lens system 25 is housed inside a lens barrel 26. A prism 27 in the shape of a right triangle and having a tilted surface as a reflection surface is disposed behind the objective lens system 25, and an optical path is bent downward by the prism 27. An imaging surface 29a of a CCD 29 is, as well known, covered by a transparent cover glass 28. The CCD 29 is disposed such that the cover glass 28 is in contact with a lower surface of the prism 27. On the imaging surface 29a of the CCD 29, an image is formed by the optical system. Note that a CMOS image sensor may be used instead of the CCD 29.

A front end 14a of the distal portion 14 is formed of a hard resin and a protective cover 31 thereof is formed of soft resin. Inside the protective cover 31 is provided a metallic tube portion 32. One end of the tube portion 32 is joined to the front end 14a.

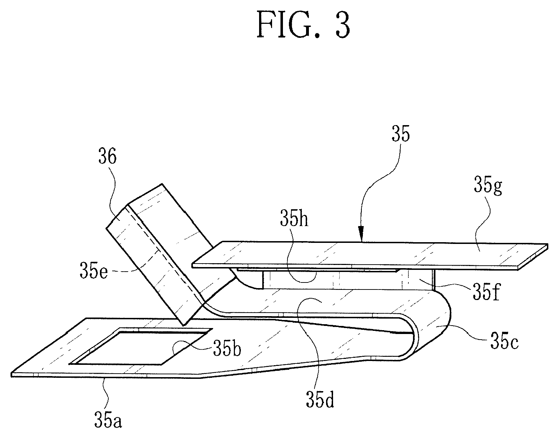

The CCD 29 is disposed such that the cover glass 28 is housed inside an opening 35b (see Fig. 3) formed on a first tip 35a of the long slender flexible substrate 35, and the CCD 29 is electrically connected to the first tip 35a by a bonding wire while a peripheral part of the CCD 29 is placed closely to an external surface of the first tip 35a. Thus, the CCD 29 is attached to the flexible substrate 35. The flexible substrate 35 has a bent portion 35c that is bent into U-shape, a straight portion 35d that extends substantially in a straight line, and a second tip 35e that reaches the vicinity of the prism 27.

A predetermined length range of the second tip 35e is bent relative to the straight portion 35d so as to be substantially parallel to the tilted surface of the prism 27. On a surface of the second tip 35e facing the prism 27 is provided components such as a circuit for driving the CCD 29, an amplifier for amplifying the video signal outputted from the CCD 29, and the like. Note that the components are virtually illustrated with broken lines. Such configuration contributes to the reduction in size of the imaging device since the components are provided near the prism 27, as compared to the case where the components are mounted on the straight portion 35d, and further contributes to reduction in size of the distal portion 14. Specifically, a diameter of the distal portion 14 may be made small, and a length thereof may be made short.

A channel-shaped cover 36 is adhered to the second tip 35e, and both side edges of the cover 36 are fixed to both side surfaces of the prism 27, respectively. The above-described amplifier and the like are covered with the cover 36 to be protected. Note that although the components such as the CCD 29 and the amplifier emit heat when driven, the heat is dissipated by the flexible substrate 35 and a connecting member 40, which is described later.

As shown in Fig. 3, the flexible substrate 35 is provided with a branch portion 35f that protrudes in a direction orthogonal to a longitudinal direction of the straight portion 35d and is bent at a right angle. A rectangular sub-substrate 35g is provided at a tip of the branch portion 35f. Surfaces of the sub-substrate 35g are orthogonal to surfaces of the branch portion 35f and substantially parallel to surfaces of the straight portion 35d. The surface of the sub-substrate 35g that faces the straight portion 35d is provided a soldering portion 35h. Each signal line 38 of a later-described signal cable 37 is connected to each of terminals provided on the soldering portion 35h.

As shown in Fig. 2, the signal cable 37 is a multi conductor cable housing the plural signal lines 38 in a cylindrical cable cover 39, and inserted through the insertion section 11, the operation section 12 and the universal cord 13.

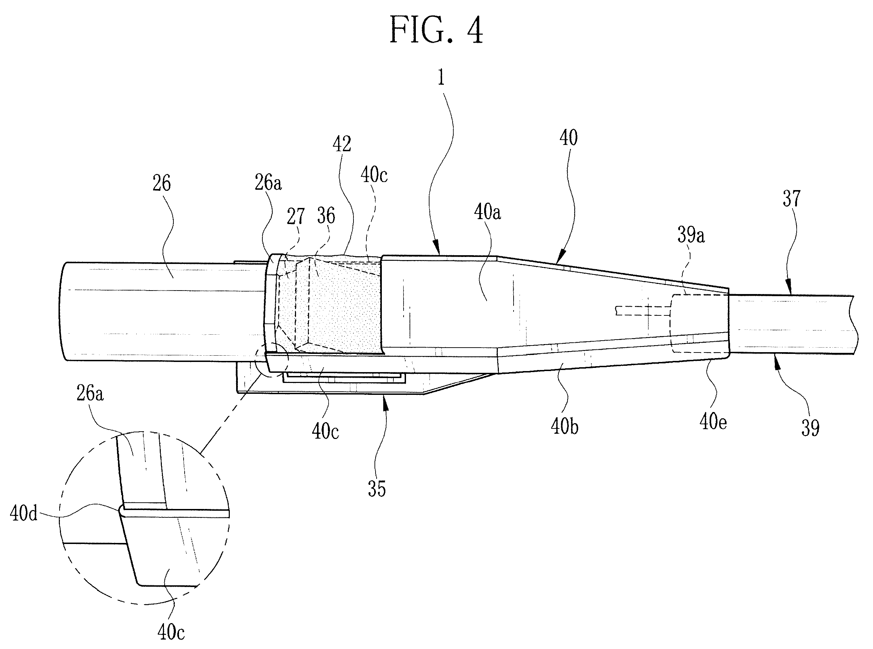

In Fig. 4 illustrating a first embodiment of the imaging device, a flange 26a that is formed at an end of the lens barrel 26 and an end portion 39a of the cable cover 39 are connected by the rigid metallic connecting member 40. The connecting member 40 is constituted of a long slender main body 40a for covering the sub-substrate 35g (see Figs. 2 and 3) and skirts 40b provided at both sides of the main body 40a. The skirts 40b are bent at substantially a right angle. Thus the connecting member 40 is formed to substantially a channel shape.

A pair of arms 40c is formed at leading ends of the skirts 40b so as to extend toward the lens barrel 26. At a tip of each arm 40c is provided a claw 40d bent inward. The claws 40d are engaged to a rim of the flange 26a. As shown in Fig. 4, rear ends 40e of the skirts 40c house the signal cable 37, namely, the end portion 39a of the cable cover 39 inside. The rear ends 40e may be fixed to the end portion 39a at both sides by an adhesive material. Alternatively, the rear ends 40e may be fixed to the end portion 39a by nipping it therebetween.

As shown in Fig. 2, a first adhesive material (first resin) 42 is filled into a hollow portion surround by the channel-shaped connecting member 40, and hardened. Owing to this, a part of the prism 27, the cover 36, a top surface (upper surface in the drawing) of the straight portion 35d, the branch portion 35f, the sub-substrate 35g, the soldering portion 35h, each signal line 38 of the signal cable 37, and the end portion 39a of the cable cover 39 are fixed inside the connecting member 40.

Moreover, a second adhesive material (second resin) 43 is filled into a hollow portion surrounded by the CCD 29, the first tip 35a, the bent portion 35c and the straight portion 35d of the flexible substrate 35, and the prism 27, that is, the hollow portion substantially inside the bent portion 35c, and hardened. The second adhesive material 43 is also filled into a hollow portion surrounded by the lens barrel 26, a part of the prism 27, the CCD 29, a rim of the cover glass 28, and the first tip 35a of the flexible substrate 35, and hardened. Further, the second adhesive material 43 is applied to a rim of the CCD 29 and an outer surface of the first tip 35a, and hardened. Owing to this, the CCD 29 and the first tip 35a of the flexible substrate 35 are not only electrically connected, but also integrated by the adhesive material 43. In addition, a part of the lens barrel 26 is adhered to the part of the prism 27, the rim of the cover glass 28, and the first tip 35a of the flexible substrate 35. Note that the second adhesive material 43 may be applied to a whole surface of the CCD 29 to seal it.

The first adhesive material 42 is preferably an epoxy-based or acrylic adhesive material with a hardness of D70 to D90 (hard type) that is measured after being hardened using a type-D durometer in accordance with JIS-K-7215. The second adhesive material 43 is preferably an epoxy-based or silicone-based adhesive material with a hardness of A30 to A100 (soft type) that is measured after being hardened with a type-A durometer in accordance with JIS-K-7215. The hardness of the first adhesive material 42 after being hardened is high (hard), and the hardness of the second adhesive material 43 is lower (softer) than the hardness of the first adhesive material 42.

Thus, the imaging device of the present invention is constituted of the objective lens system 25, the prism 27, the CCD 29, and the flexible substrate 35 as main components. In addition, the imaging device auxiliary includes the lens barrel 26, the cover 36, the connecting member 40, the first adhesive material 42, and the second adhesive material 43.

The imaging device 1 configured as such is fixed at a predetermined position inside the front end 14a of the distal portion 14 by screwing male threads 45 into female threads provided at, for example, three points on an outer circumference of the lens barrel 26. Thereafter, the tube portion 32 is fixed to a rear portion of the front end 14a, and the tube portion 32 and the rear portion of the front end 14a are covered with the protective cover 31.

The end portion 39a of the cable cover 39 is adhered to the inside of the rear ends 40e of the connecting member 40 by the first adhesive material 42. The lens barrel 26 is fixed to the front end 14a, and the pair of the claws 40d of the connecting member 40 is engaged to the flange 26a. Owing to this, each of the signal lines 38 of the signal cable 37 do not move even when the signal cable 37 is pulled into a direction away from the connecting member 40 by the bending and the like of the bending portion 15. Therefore, each of the signal lines 38 is neither peeled from each terminal of the soldering portion 35h nor broken.

Moreover, since the sub-substrate 35g of the flexible substrate 35 is adhered and fixed to the connecting member 40 by the first adhesive material 42, the distance between each terminal of the soldering portion 35h and each signal line 38 is not lessened even when the signal cable 37 is pushed toward the connecting member 40 due to the bending and the like of the bending portion 15. Therefore, each of the signal lines 38 is neither peeled from each terminal of the soldering portion 35h nor broken.

Thus, each of the signal lines 38 is neither peeled from each terminal of the soldering portion 35h nor broken even when the signal cable 37 is pushed and pulled. There may be a possibility that the components as a whole being adhered by the first adhesive material 42 (the connecting member 40 and the parts thereinside) are under load when the signal cable 37 is strongly pushed and pulled. However, except the flexible substrate 35, the part adhered by the first adhesive material 42 and the part adhered by the second adhesive material 43 are independent from each other across the straight portion 35d of the flexible substrate 35 as a border line. Moreover, since the second adhesive material 43 is relatively soft, it permits deformation of the flexible substrate 35 to some degree. Therefore, the load applied to the part adhered by the first adhesive material 42 is compromised by the deformation of the flexible substrate 35, especially the bent portion 35c, and therefore not affecting the part adhered by the second adhesive material 43. Accordingly, the CCD 29, which is a most important component, is not loaded, and therefore the CCD 29 is not damaged.

When forces of strong pushing and pulling, shaking, and twisting are applied to the connecting member 40, the connecting member 40 may be deformed. In this case, the first adhesive material 42 follows the deformation of the connecting member 40 while maintaining a certain shape. When the first adhesive material 42 is deformed following the deformation of the connecting member 40, the straight portion 35d of the flexible substrate 35 is deformed following the deformation of the first adhesive material 42. However, since the second adhesive material 43 is softer than the first adhesive material 42, the degree of the deformation is kept small around the CCD 29, and the forces applied to the CCD 29 are made small.

Since the CCD 29 and the first tip 35a of the flexible substrate 35 are not covered with the connecting member 40, in other words, exposed outside the connecting member 40, the diameter of the distal portion 14 can be made smaller. In addition, even when the connecting member 40 is deformed, the forces caused by the deformation of the connecting member 40 are not applied to the CCD 29 since the CCD 29 is outside the connecting member 40.

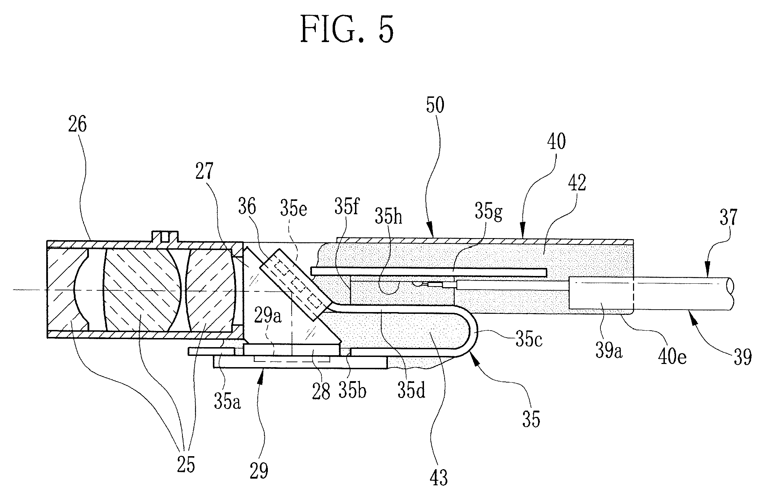

Next, an imaging device 50 according to a second embodiment of the present invention is explained with reference to Fig. 5. In the second embodiment, the same reference numbers as those of the first embodiment indicate components having the same function and structure as those of the first embodiment, and detailed description thereof will be omitted (same applied in the following embodiments). In the imaging device 50, the area adhered by the first adhesive material 42 is up to the cover 36, and the prism 27 is not adhered by the first adhesive material 42. Besides this, the configuration of the imaging device 50 is same as the configuration of the imaging device 1 of the first embodiment.

In the second embodiment, when the signal cable 37 is pushed toward the connecting member 40, although each of the signal lines 38 is neither peeled from each terminal of the soldering portion 35h nor broken, there may be a possibility that the connecting member 40 is moved toward the lens barrel 26. However, the move of the connecting member 40 does not affect the electric and mechanical connections between the CCD 29 and the flexible substrate 35 since the lens barrel 26 and the first tip 35a of the flexible substrate 35 are adhered by the second adhesive material 43, and the move of the connecting member 40 toward the lens barrel 26 is absorbed by the deformation of the bent portion 35c and does not reach the first tip 35a. In the second embodiment, the amount of the first adhesive material 42 used can be reduced as compared to the first embodiment, which contributes to the reduction in cost of the imaging device.

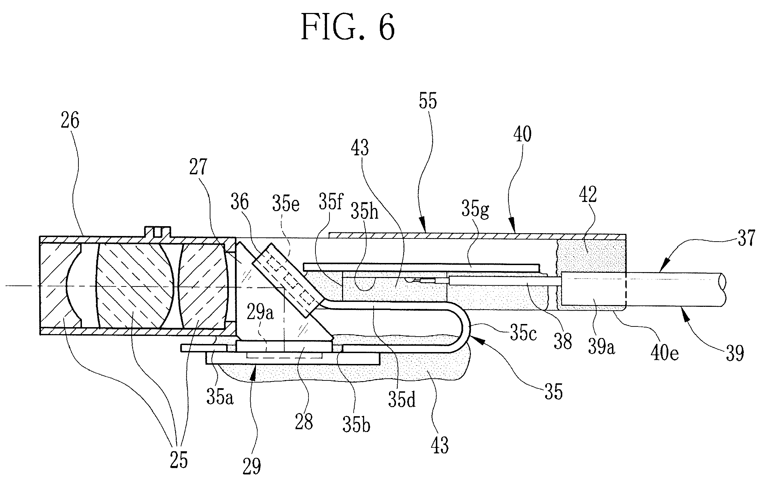

Next, an imaging device 55 according to a third embodiment of the present invention is explained with reference to Fig. 6. In the imaging device 55, the first adhesive material (first resin) 42 is filled inside the rear ends 40e, and the second adhesive material (second resin) 43 is filled between the straight portion 35d and the sub-substrate 35g. Moreover, the second adhesive material (corresponding to third resin in claim 4) 43 is applied to the CCD 29, the cover glass 28 and the first tip 35a of the flexible substrate 35, and hardened. Besides this, the configuration of the imaging device 55 is same as the configuration of the imaging device 1 of the first embodiment.

In the third embodiment, when the signal cable 37 is pushed toward the connecting member 40, there may be a possibility that the connecting member 40 is moved toward the lens barrel 26. However, similarly to the second embodiment, the move of the connecting member 40 does not affect the electric and mechanical connections between the CCD 29 and the flexible substrate 35 since the move of the connecting member 40 toward the lens barrel 26 is absorbed by the deformation of the bent portion 35c and does not reach the first tip 35a.

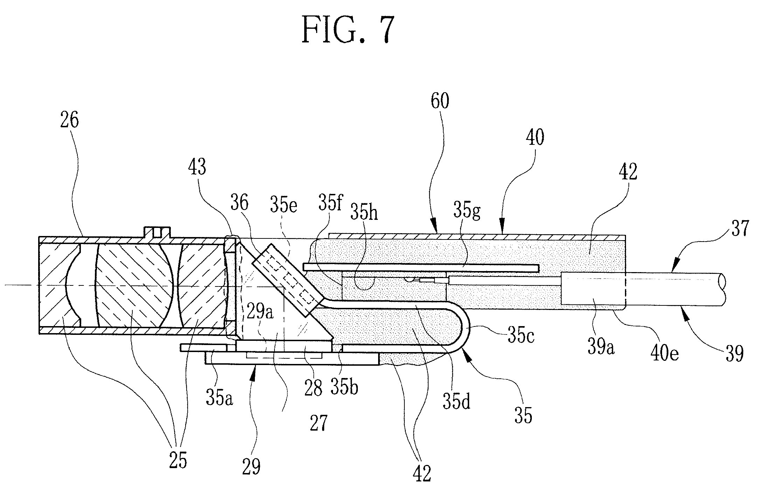

Next, an imaging device 60 according to a fourth embodiment of the present invention is explained with reference to Fig. 7. In the imaging device 60, the first adhesive material 42 is filled into the hollow portion surrounded by the connecting member 40 except the vicinity of the prism 27 and into the hollow portion inside the bent portion 35c, and hardened. Moreover, the first adhesive material (first resin) 42 is applied to the rim of the CCD 29 and the outer surface of the first tip 35a, and hardened. Further, the second adhesive material 43 is applied to the outer circumference of the lens barrel 26 where the flange 26a and the prism 27 are in contact with each other including the part where the pair of the claws 40d is engaged to the flange 26a of the lens barrel 26 (see Fig. 4), and hardened.

In the fourth embodiment, the distance between the lens barrel 26 and the signal cable 37 does not change when the signal cable 37 is pushed and pulled. Owing to this, the soldering of each signal line 38 to each terminal of the soldering portion 35h does not come off, and the connection between the CCD 29 and the flexible substrate 35 is not peeled. If a big impact is caused to the distal portion 14 by, for example, dropping the electronic endoscope 10, the impact force is applied in a direction orthogonal to the longitudinal direction of the connecting member 40, which may cause peeling of the connection between the flange 26a of the lens barrel 26 and the prism 27 made by the second adhesive material 43 having relatively low adhesion. However, if the connection between the lens barrel 26 and the prism 27 is peeled, the impact force which the lens barrel 26 and the prism 27 receive is weakened, and therefore preventing the breakage of the lens barrel 26 and the prism 27. When the connection between the lens barrel 26 and the prism 27 is peeled, the second adhesive material 43 is removed completely, and the second adhesive material 43 is applied again to be hardened. In this way, a repair can be made with ease at low cost.

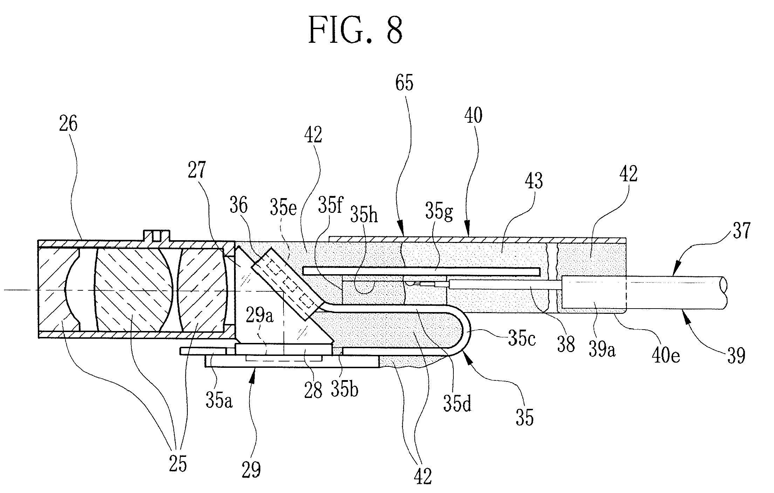

Next, in an imaging device 65 according to fifth embodiment of the present invention shown in Fig. 8, the first adhesive material (first resin) 42 is filled inside the rear ends 40e. In the hollow portion surrounded by the connecting member 40 except the rear ends 40e, the second adhesive material (second resin) 43 is filled in the area from where each signal line 38 of the signal cable 37 is soldered to each terminal of the soldering portion 35h to the edge on the side of the rear ends 40e, and the second adhesive material 43 is filled in the rest of the hollow portion on the side of the prism 27, and hardened. In addition, the first adhesive material 42 is filled in the hollow portion inside the bent portion 35c, and hardened. Moreover, the first adhesive material 42 is applied to the rim of the CCD 29 and the outer surface of the first tip 35a, and hardened.

In the fifth embodiment, when the signal cable 37 is pushed and pulled, the force thereof is not applied to the prism 27 and the CCD 29 to damage them. Even if a big force is applied to the signal cable 37 or the connecting member 40, and the second adhesive material 43 having relatively low adhesion is peeled and the soldering of each signal line 38 of each terminal of the soldering portion 35h is peeled, a repair can be made with ease at low cost by removing the second adhesive material 43 completely and soldering again, and applying the second adhesive material 43 to be hardened.

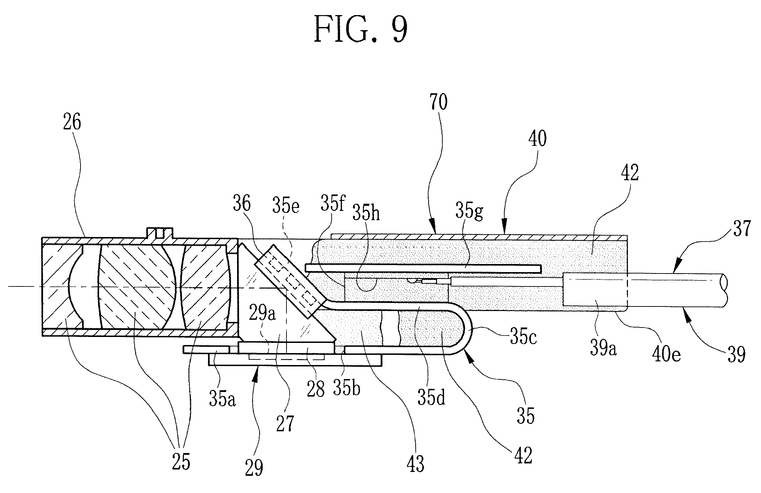

Next, an imaging device 70 according to sixth embodiment of the present invention is shown in Fig. 9. In the imaging device 70, the first adhesive material (first resin) 42 is filled into the hollow portion surrounded by the connecting member 40 except the vicinity of the prism 27 and into the hollow portion inside the bent portion 35c except the vicinity of the prism 27, and hardened. The second adhesive material (second resin) 43 is filled into the hollow portion inside the bent portion 35c on the side of the prism 27, and hardened.

In the sixth embodiment, the prism 27, the CCD 29 and the flexible substrate 35 are adhered by the second adhesive material 43 having relatively low adhesion. Therefore, when a big impact is caused to the distal portion 14, the flexible substrate 35 may move, which may cause peeling of the second adhesive material 43. However, owing to the peeling of the second adhesive material 43, the impact force is not transmitted to the prism 27 or the CCD 29. Therefore, the breakage of the prism 27 or the CCD 29 can be prevented.

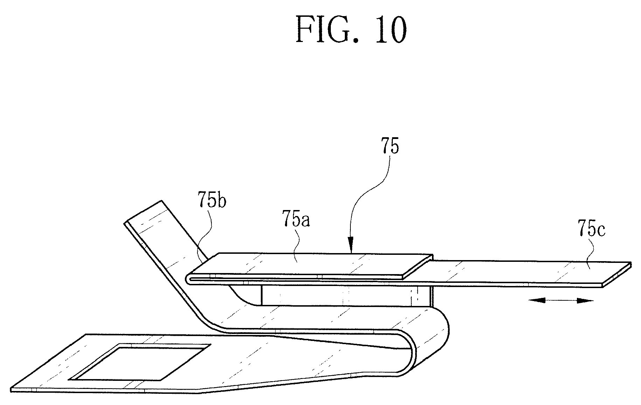

A flexible substrate 75 shown in Fig. 10 has a sub-substrate 75a like the flexible substrate 35. The sub-substrate 75a has a length in its longitudinal direction longer than the flexible substrate 35, and is folded inward at a folding portion 75b. A front end 75c of the sub-substrate 75 extends to the side of the signal cable 37 (see Fig. 2). The soldering portion is provided on a bottom surface (lower surface in the drawing) of the extending front end 75c, and each signal line 38 of the signal cable 37 is soldered to each terminal of the soldering portion. The load applied to the flexible substrate 75 by pushing and pulling the signal cable 37 is absorbed by the deformation of the folded portion 75b.

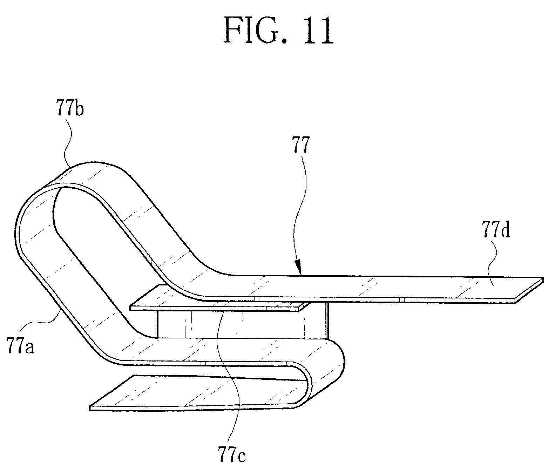

Moreover, in a flexible substrate 77 shown in Fig. 11, an extending portion 77a corresponding to the second tip 35e of the flexible substrate 35 extends longer than the second tip 35e to the side of the signal cable 37 (see Fig. 2) via a curbed portion 77b and over a top surface of a sub-substrate 77c. The soldering portion is provided on a bottom surface (lower surface in the drawing) of a front end 77d, and each signal line 38 of the signal cable 37 is soldered to each terminal of the soldering portion. The load applied to the flexible substrate 77 by pushing and pulling the signal cable 37 is absorbed by the deformation of the curbed portion 77b.

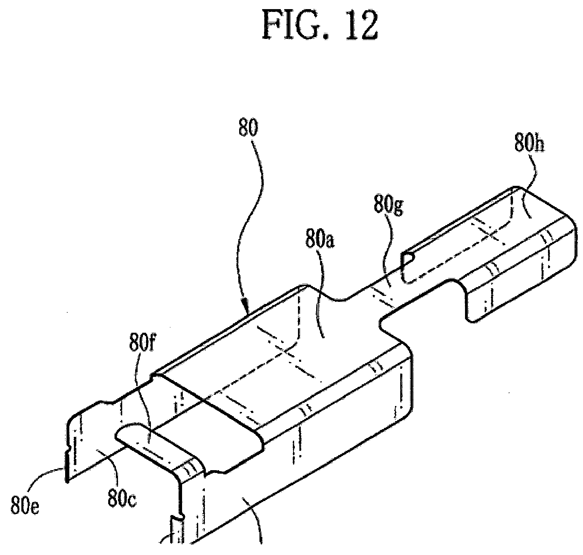

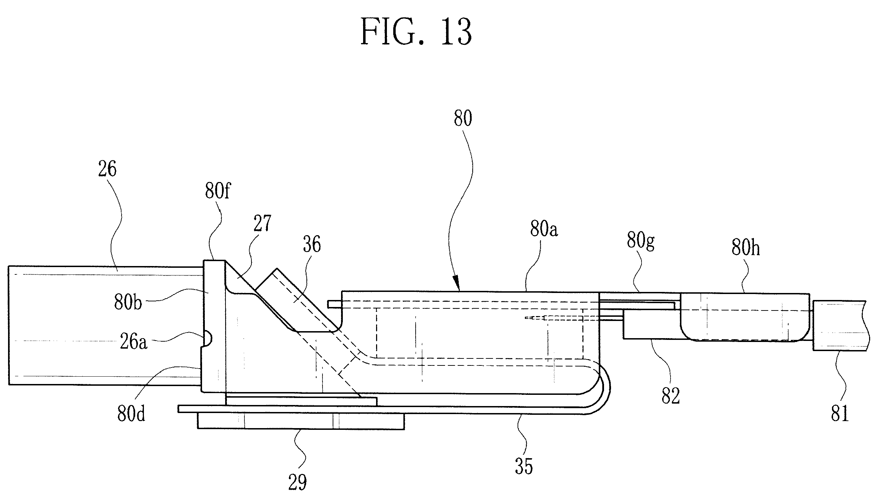

Next, an example of another connecting member is explained. As shown in Figs. 12 and 13, a connecting member 80 has a channel-shaped main body 80a, and a pair of arms 80b, 80c is formed at a leading end thereof being engaged to the lens barrel 26, similarly to the connecting member 40. At a tip of each arm 80b, 80c is provided a claw 80d, 80e bent inward. A retention piece 80f for pressing the flange 26a down is formed to be integrated with the arm 80b. Owing to the retention piece 80f, the engagement with the flange 26a is made tight. At a rear end of the main body 80a is integrally formed a rear end portion 80h having a channel shape smaller than the main body 80a via a narrow portion 80g having a width narrower than the main body 80a.

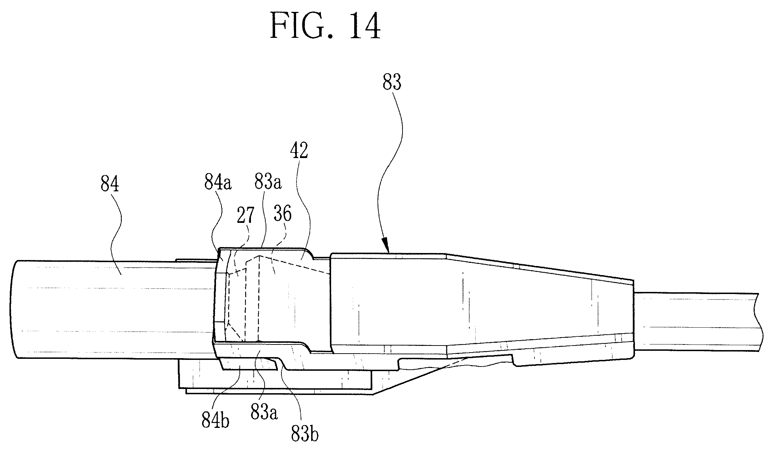

A connecting member 83 shown in Fig. 14 has a pair of cranked arms 83a in which the pair of arms 83a is bent upward at the middle of the arms 83a. Moreover, the prism 27 and the cover 36 are not protruded outside of the pair of arms 83a. For this configuration, the prism 27 and the cover 36 are completely covered with the first adhesive material 42 when the first adhesive material 42 is filled between the arms 83a, which is advantageous in protecting the prism 27 and the cover 36. Note that a lug 84b of a flange 84a of a lens barrel 84 is preferably engaged to a recess 83b of each arm 83a.

In the above embodiments, the end portion of the signal cable is fixed to the inside of the connecting member by the adhesive material being filled. It is also possible to fix the end of the signal cable to the connecting member by swaging the end portion of the connecting member. In this case, a protective tube may be further covered over the cable cover so that the signal cable is not damaged by swaging the end portion of the connecting member.

Although the present invention has been fully described by the way of the preferred embodiment thereof with reference to the accompanying drawings, various changes and modifications will be apparent to those having skill in this field. Therefore, unless otherwise these changes and modifications depart from the scope of the present invention, they should be construed as included therein.

Preferred embodiments of the invention are outlined in the following paragraphs:
1. An imaging device (1) including a lens barrel (26) housing an objective lens system (25), a prism (27) disposed behind the lens barrel (26), and a solid state imaging element (29) disposed on an optical path that is bent by the prism (27), said solid state imaging element (29) photoelectrically converting an image formed by said objective lens system (25), said imaging device (1) comprising:
   a flexible substrate (35) on which said solid state imaging element (29) is mounted at a first tip (35a);
   a signal cable (37) electrically connected to said flexible substrate (35); and
   a connecting member (40) for connecting said lens barrel (26) and said signal cable (37), said connecting member (40) not covering at least one surface of said prism (27), said solid state imaging element (29), and said first tip (35a) of said flexible substrate (35).
2. The imaging device (1) according to para. 1, wherein said prism (27) is in the shape of a right triangle having a tilted surface as a reflection surface, said one surface being said tilted surface.
3. The imaging device (1) according to para. 1, wherein said connecting member (40) has a channel-like portion for covering at least an end portion of said signal cable (37) and a part of said flexible substrate (35) from three sides, said end portion of said signal cable (37) being fixed to a narrow end (40e) of said channel-like portion.
4. The imaging device (1) according to para. 3, further comprising:
   a first resin (42) being filled inside said channel-like portion of said connecting member (40) for sealing at least a part of said prism (27) and a part of said flexible substrate (35); and
   a second resin (43) for sealing said prism (27) and a part of said flexible substrate (35) except the parts being sealed by said first resin (42).
5. The imaging device (1) according to para. 4, wherein said second resin (43) is applied to at least a periphery of said solid state imaging element (29).
6. The imaging device (1) according to para. 3, further comprising:
   a first resin (42) for sealing said end portion of said signal cable (37);
   a second resin (43) for sealing a part of said flexible substrate (35) where said signal cable (37) is connected; and
   a third resin (43) for sealing a part of said flexible substrate (35) except the parts being sealed by said first resin (42) and said second resin (43) and a part of said prism (27).
7. The imaging device (1) according to para. 4, wherein after being hardened, the hardness of said second resin (43) is lower than the hardness of said first resin (42).
8. The imaging device (1) according to para. 6, wherein after being hardened, the hardness of said second resin (43) and said third resin (43) are almost equal and is lower than the hardness of said first resin (42).
9. The imaging device (1) according to para. 3, further comprising:
   a first resin (42) for sealing said end portion of said signal cable (37);
   a second resin (42) for sealing a part of said prism (27) and a part of said flexible substrate (35) where said signal cable (37) is connected;
   a third resin (42) for sealing a part of said flexible substrate (35) except the parts being sealed by said first resin (34) and said second resin (42); and
   a fourth resin (43) for sealing a periphery of a part where said lens barrel (26) and said prism (27) are in contact.
10. The imaging device (1) according to para. 9, wherein after being hardened, the hardness of said first resin (42), said second resin (42) and said third resin (42) are equally high, and the hardness of said fourth resin (43) is lower than the hardness of said first resin (42), said second resin (42) and said third resin (42).
11. The imaging device (1) according to para. 3, further comprising:
   a first resin (42) for sealing said end portion of said signal cable (37);
   a second resin (43) for sealing a part of said flexible substrate (35) where said signal cable (37) is connected;
   a third resin (42) for sealing a part of said prism (27), a part of said lens barrel (26) near said prism (27) and a part of said flexible substrate (35) except the part being sealed by said second resin (43); and
   a fourth resin (42) for sealing a part of said prism (27) and a part of said flexible substrate (35) except the parts being sealed by said second resin (43) and said third resin (42).
12. The imaging device (1) according to para. 11, wherein after being hardened, the hardness of said second resin (43) is lower than the hardness of said first resin (42), and the hardness of said third resin (42) and said fourth resin (42) are almost equal to the hardness of said first resin (42).
13. An electronic endoscope (10), comprising an insertion section (11) in which said imaging device (1) according to para. 1 is incorporated at a distal portion (14) of said insertion section (11).

## Claims

1. An electronic endoscope (10) comprising:
a solid state imaging element (29) adapted to photoelectrically convert an optical image from a lens barrel (26), the optical image being focused on the solid state imaging element (29) through a prism (27);
a flexible substrate (35) electrically connected to the solid state imaging element (29) and mounted with electronic circuits, the flexible substrate (35) being folded through a branch portion (35f) so as to have two or more surfaces overlaid in a direction perpendicular to a longitudinal direction of the electronic endoscope (10);
a signal cable (37) electrically connected to the flexible substrate (35) and adapted to transmit an image signal; and
a channel-shaped member (40, 80) engaged to the lens barrel (26).

2. The electronic endoscope (10) according to claim 1, wherein the lowermost surface of the flexible substrate (35) is connected to a light incident side surface of the solid state imaging element (29).

3. The electronic endoscope (10) according to claim 1 or 2, wherein a first adhesive material (42) is filled inside the flexible substrate (35).

4. The electronic endoscope (10) according to claim 1 or 2, wherein a first adhesive material (42) is filled inside the channel -shaped member (40, 80).

5. The electronic endoscope (10) according to claim 3 or 4, wherein the first adhesive material (42) integrally seals from the vicinity of the side surfaces of the prism (27) to a connecting portion between the flexible substrate (35) and the signal cable (37).

6. An electronic endoscope (10) comprising:
a solid state imaging element (29) adapted to photoelectrically convert an optical image from a lens barrel (26), the optical image being focused on the solid state imaging element (29) through a prism (27);
a flexible substrate (35) electrically connected to the solid state imaging element (29) and mounted with electronic circuits, the flexible substrate (35) being folded through a branch portion (35f) so as to have two or more surfaces overlaid in a direction perpendicular to a longitudinal direction of the electronic endoscope (10);
a signal cable (37) electrically connected to the flexible substrate (35) and adapted to transmit an image signal; and
a channel-shaped member (40, 80) for covering top and side surfaces of the prism (27).
